# FASCICULE DE BREVET EUROPEEN

(11) **EP 3 472 298 B1**
(45) Date de publication et mention de la délivrance du brevet: **08.11.2023**
(21) Numéro de dépôt: 17737310.7
(22) Date de dépôt: 13.06.2017
(51) Int. Cl.: C12G 1/022, C12G 3/02, C12M 1/00

(54) **PROCÉDÉ DE PRÉPARATION D'UN VIN À FAIBLE DEGRÉ ALCOOLIQUE**
VERFAHREN ZUR HERSTELLUNG VON WEIN MIT GERINGEM ALKOHOLGEHALT
METHOD FOR PRODUCING WINE WITH A LOW ALCOHOL CONTENT

(30) Priorité: 16.06.2016 FR 1600961
(43) Date de publication de la demande: 24.04.2019
(73) Titulaire: AB7 Industries S.A., 31450 Deyme (FR)
(72) Inventeur: CHELLE, René, 31450 Deyme (FR)
(86) Numéro de dépôt international: PCT/FR2017/000118
(87) Numéro de publication internationale: WO 2017/216431

(56) Documents cités:
- WO-A1-2014/114448
- WO-A1-2014/114448
- CN-A- 103 897 918
- CN-A- 103 897 918
- FR-A1- 2 887 257
- FR-A1- 2 887 257

## Description

La présente invention se rapporte à la fermentation de boissons alcoolisées à partir d'un jus de raisin.

Elle a pour objet un procédé de préparation d'un vin à degré d'alcool réduit faisant appel uniquement à des méthodes biologiques de fermentation de jus de fruit naturel. Ce procédé permet notamment de produire, à partir d'un moût dont le potentiel alcoolique P est communément de 8° à 15°, une boisson ayant un taux d'alcool inférieur d'au moins 4° à son potentiel alcoolique P.

On sait que la lutte contre l'alcoolisme est un enjeu important de santé publique. Cependant, les vins sans alcool, qui ont été proposés jusqu'à ce jour, n'ont pas été accueillis favorablement par les consommateurs, en raison du fait qu'ils ont des caractéristiques gustatives peu développées. Ces boissons sont souvent obtenues par fermentation de la totalité des sucres présents dans le jus de raisin, puis extraction d'une partie de l'alcool formé par des méthodes physiques (voir notamment les brevets EP-A 0193206 ou EP-A 228572) ; dans ce cas, leur production nécessite donc au moins une étape supplémentaire qui correspond à une augmentation du coût. En outre, il semble que cette extraction élimine ou dégrade la plupart des composés responsables des propriétés et qualités organoleptiques du vin.

La présente invention propose un procédé faisant appel uniquement à des méthodes biologiques de fermentation en mettant en oeuvre une culture de levures ; ce procédé est fondé sur des mécanismes de fermentation identiques à ceux qui sont communément employés pour la production des vins. Le vin obtenu possède des qualités organoleptiques attractives pour le consommateur.

Certains producteurs ont déjà proposé un procédé (voir EP 0440975) comportant différentes étapes sous aération continue plus ou moins contrôlée afin d'obtenir un vin à teneur réduite en alcool. Dans ce procédé, on propose de faire une première étape de fermentation alcoolique d'une fraction de jus de raisin sous apport limité en oxygène, ceci conduisant à une production d'alcool ; cette étape est alors suivie d'un traitement aérobie après l'ajout d'une seconde fraction de jus. Dans ce procédé, l'alcool produit dans la première étape devient un substrat et, est en fait consommé par les levures au cours de la deuxième étape, ce qui réduit considérablement le rendement de l'ensemble. En outre, le renouvellement de la population de levures de fermentation n'est plus assuré après consommation totale du sucre au cours de la deuxième étape. Ce procédé ne donne donc pas satisfaction.

Le brevet CN103897918 décrit un fermenteur aérobie mais ne décrit pas une méthode pour obtenir un vin à faible teneur en alcool.

Dans le brevet français 2887257, on a proposé un procédé qui comprend essentiellement les étapes consistant à :
a. alimenter un réacteur de culture aérobie contenant des levures en phase de multiplication, avec une première fraction de jus de raisin ;
b. alimenter un réacteur de fermentation anaérobie avec le milieu chargé en levures obtenues à l'étape a et une seconde fraction de jus de raisin pour obtenir un moût ;
c. filtrer le moût pour séparer les levures et la boisson fermentée obtenue. Dans une variante, le sucre de la première fraction est consommé en totalité ou en quasi-totalité dans le réacteur de culture. Dans ce procédé, le premier réacteur de culture entraine la multiplication des levures qui sont introduites et ce premier réacteur alimente le deuxième réacteur, à savoir, un réacteur de fermentation, qui fonctionne en anaérobie, de sorte que les levures consomment leurs nutriments et notamment l'alcool et le sucre qui se trouvent dans le milieu contenu dans le premier réacteur ; dans le deuxième réacteur, la grande quantité de levures permet d'obtenir un faible taux d'alcool ; et, de la sorte, la sortie du deuxième réacteur étant filtrée pour séparer les levures, constitue la boisson fermentée à faible degré d'alcool désirée.

Il existe donc un besoin de fournir un dispositif simplifié destiné à la fabrication, en continu, d'un vin à faible degré alcoolique ayant de bonnes qualités organoleptiques par le concours d'un seul réacteur spécifique capable de désucrer le moût grâce aux levures dont la prolifération au sein dudit réacteur est optimisée. De même, il existe un besoin de fournir un procédé dont la mise en oeuvre permet de diluer progressivement le taux de sucre d'un moût initial à traiter tout en assurant un redémarrage rapide de la vinification.

Selon la présente invention, on a constaté qu'il était possible de simplifier de façon significative le dispositif proposé par le brevet EP 1891199 tout en conservant le principe selon lequel on effectue le désucrage du moût sur une fraction de traitement que l'on remélange ensuite avec la fraction non traitée que l'on laisse subsister dans la cuve de vinification. Par ailleurs, le procédé selon l'invention permet, dans une variante, d'obtenir une grande quantité de levures que l'on peut utiliser pour ensemencer des cuves et pour améliorer les qualités organoleptiques du vin à faible degré alcoolique ; on sait, en effet, que l'on peut améliorer le goût du vin en fragilisant les parois cellulaires des levures, ce qui conduit à la mise à disposition de polysaccharides ou de divers composés aromatiques, lesquels contribuent en tant que substrats, par exemple, au démarrage de la fermentation malolactique. En mettant en oeuvre l'invention, on peut valoriser les levures « fraiches » que produit le procédé selon l'invention alors qu'à ce jour, les producteurs de vins achetaient des levures déshydratées dont la conservation est limitée dans le temps (5 à 8 semaines environ) et qui, au surplus, sont vendues sous forme de poudre mélangée à divers débris cellulaires.

La présente invention a, en conséquence, pour objet un procédé de préparation d'un vin ayant de bonnes qualités organoleptiques et un faible degré d'alcool d à partir d'un moût de raisin, dont le taux de sucre élevé conduirait, après un traitement de fermentation classique, à un vin de degré alcoolique potentiel D > d, ledit moût se trouvant dans une cuve de vinification à partir de laquelle on prélève une aliquote de moût dans un système de fermentation mettant en oeuvre des levures, ledit système comportant deux étapes de fermentation, dont l'une est aérobie et l'autre est anaérobie, le flux de sortie de la première étape alimentant la deuxième étape, qui fournit l'aliquote traitée et la renvoie dans la cuve de vinification pour y être mélangée avec le moût initial restant, caractérisé en ce que, dans un fermenteur-désucreur unique, on assure simultanément l'homogénéisation du milieu de fermentation et l'aération des levures, dont la multiplication en aérobie consomme du sucre sans produire d'éthanol, au moyen d'une circulation d'air associée à un flux circulant de l'aliquote provenant de la cuve de vinification, ladite homogénéisation s'effectuant avec un chauffage régulé, qui amène la température de l'aliquote à une valeur correspondant à une fermentation appropriée en mode aérobie jusqu'à une consommation complète ou quasi complète du sucre de l'aliquote de moût traité, et que l'on transfère l'aliquote de traitement désucré depuis le fermenteur/désucreur vers un décanteur muni d'un moyen de refroidissement pour obtenir la clarification souhaitée du moût.

Dans un mode préféré de mise en oeuvre du procédé ci-dessus défini, le flux circulant de l'aliquote de moût en cours de traitement et la circulation d'air associée sont effectués de bas en haut, parallèlement à la ligne moyenne du fermenteur-désucreur. Selon cette variante, le fermenteur-désucreur fonctionne selon un mode connu, qui est généralement appelé « air lift ».

Dans un mode de mise en oeuvre du procédé selon l'invention, le chauffage du fermenteur-désucreur maintient, dans la fraction du moût en cours de traitement qu'il contient, une température sensiblement homogène comprise entre 30 et 45°C et de préférence, voisine de 40°C.

Selon un mode de mise en oeuvre du procédé selon l'invention, l'aliquote de moût désucré réintroduite dans la cuve de vinification contient des levures, et provient du fermenteur-désucreur, afin d'ensemencer le moût restant dans ladite cuve pour assurer un redémarrage plus rapide de la vinification.

Selon un mode de mise en oeuvre du procédé selon l'invention, on peut utiliser le chauffage du fermenteur-désucreur dans une phase de nettoyage ou de désinfection du fermenteur-désucreur pour chauffer à ébullition une quantité d'eau de lavage, qui, après condensation, est évacuée par une vanne de vidange.

Selon un mode de mise en oeuvre du procédé selon l'invention, on mesure de façon continue, au moyen d'un réfractomètre, le taux de sucre de l'aliquote de moût en cours de traitement, qui circule dans le fermenteur-désucreur, pour réguler automatiquement, par action sur l'alimentation en air, le mode aérobie de la fermentation.

Selon un mode de mise en oeuvre du procédé selon l'invention, on repère, par la mesure du taux de sucre de l'aliquote en cours de traitement dans le fermenteur-désucreur, le moment où tout le sucre de ladite fraction a été consommé par la fermentation aérobie et qu'on commande alors l'arrêt immédiat de l'entrée d'air ainsi que le chauffage dans le fermenteur-désucreur, pour écarter toute détérioration des qualités organoleptiques de ladite aliquote.

Selon un mode de mise en oeuvre du procédé selon l'invention, on mesure la densité microbienne, donc la croissance des levures, dans la fraction de traitement, au moyen d'un photomètre et l'on contrôle ainsi de manière complémentaire la fermentation dans le fermenteur-désucreur.

Selon l'invention, on transfère l'aliquote en cours de traitement désucrée depuis de fermenteur-désucreur vers un décanteur muni d'un moyen de refroidissement, pour obtenir la clarification souhaitée du moût. Dans cette mise en oeuvre du procédé, on recueille, dans le décanteur, tout ou partie des levures ayant sédimentées sous forme d'une crème dense concentrée en levures et on réintroduit lesdites levures dans la cuve de vinification, pour assurer, au moins partiellement, l'ensemencement de ladite cuve. Selon un autre mode de mise en oeuvre, on améliore les qualités organoleptiques du vin à faible degré d'alcool, en y injectant au moins une partie de la crème dense de levures récupérée dans le décanteur, après avoir traité lesdites levures, soit par traitement thermique, soit par digestion enzymatique partielle, afin d'assurer l'élevage du vin.

Un fermenteur-désucreur permettant la mise en oeuvre du procédé ci-dessus défini est également décrit. Le fermenteur décrit ci-dessous ne fait pas partie de l'invention. Un tel fermenteur comporte une enveloppe cylindrique et un conduit, qui est disposé sensiblement coaxialement à l'intérieur de l'enveloppe et qui est maintenu par rapport à elle, le conduit étant ouvert à ses deux extrémités, alors que l'enveloppe cylindrique est fermée à sa partie inférieure, pour maintenir l'aliquote de moût à traiter, un moyen chauffant régulé étant porté par l'enveloppe et coopérant au mouvement du flux circulant de l'aliquote dans le conduit, l'extrémité inférieure du conduit comportant une insufflation d'air alors que la partie haute du conduit débouche librement dans l'enveloppe.

Dans un mode préféré de réalisation, le fermenteur-désucreur comporte un moyen chauffant régulé, qui est avantageusement choisi dans le groupe formé par une plaque chauffante formant la partie inférieure de l'enveloppe, une virole chauffante entourant la paroi inférieure de l'enveloppe et l'association de ces deux types d'éléments.

Dans un mode de réalisation du fermenteur-désucreur l'enveloppe et le conduit sont des cylindres coaxiaux d'axe sensiblement vertical, la section de passage dans le conduit étant sensiblement égale à la section de passage annulaire entre le conduit et l'enveloppe, de sorte que la vitesse de déplacement de l'aliquote du moût en cours de traitement de part et d'autre de la paroi du conduit reste sensiblement constante, le niveau supérieur de l'aliquote du moût étant au-dessus de l'extrémité haute du conduit, le moût s'écoulant vers le bas dans le passage annulaire et vers le haut dans le conduit.

Dans un mode de réalisation, le remplissage du fermenteur-désucreur s'effectue au moyen d'une pompe, qui prélève, dans la cuve de vinification, une aliquote de moût à traiter et la transfère dans le fermenteur-désucreur jusqu'à ce que le niveau du moût de l'aliquote arrive au-dessus de l'extrémité haute du conduit, ce niveau étant repéré sur l'enveloppe par une détection de niveau haut pour permettre un pilotage automatique du fonctionnement.

Dans un mode de réalisation du fermenteur-désucreur on prévoit le vidage du fermenteur-désucreur dans un décanteur au moyen d'une pompe, dont le fonctionnement est géré au moyen d'un détecteur de niveau bas positionné à la même hauteur qu'une vanne de soutirage située sur la partie inférieure de l'enveloppe du fermenteur-désucreur.

Dans un mode de réalisation du fermenteur-désucreur le moyen chauffant du fermenteur-désucreur est associé à au moins une sonde thermique régulant la température de l'aliquote du moût en cours du traitement pour éviter une surchauffe locale du moût.

Dans un mode de réalisation, le fermenteur-désucreur est équipé, dans son passage annulaire, au niveau de la zone médiane du conduit, d'un réfractomètre pour la mesure continue du taux de sucre de l'aliquote de moût circulant.

Dans un mode de réalisation du fermenteur-désucreur la partie haute de l'enveloppe porte un appareil de dévésiculation, qui reçoit les gaz, vapeurs ou vésicules émanant de l'enveloppe pour récupérer au mieux les matières liquides ou liquéfiables faisant partie intégrante de l'aliquote. Dans cette réalisation, la paroi de l'appareil de dévésiculation est avantageusement maintenue sur l'enveloppe par des fixations amovibles et comporte un moyen de refroidissement, par exemple une virole externe.

Dans un mode de réalisation où le fermenteur-désucreur coopère avec un décanteur, le décanteur comporte un moyen de refroidissement pour amener l'aliquote de moût en cours de traitement à une température comprise entre 10 et 20°C pendant un temps suffisant pour atteindre la clarification souhaitée avant le renvoi de ladite aliquote dans la cuve de vinification.

Pour mieux faire comprendre l'objet de l'invention, on va en décrire ci-après, un mode de mise en oeuvre représenté sur le dessin annexé.

Sur ce dessin :

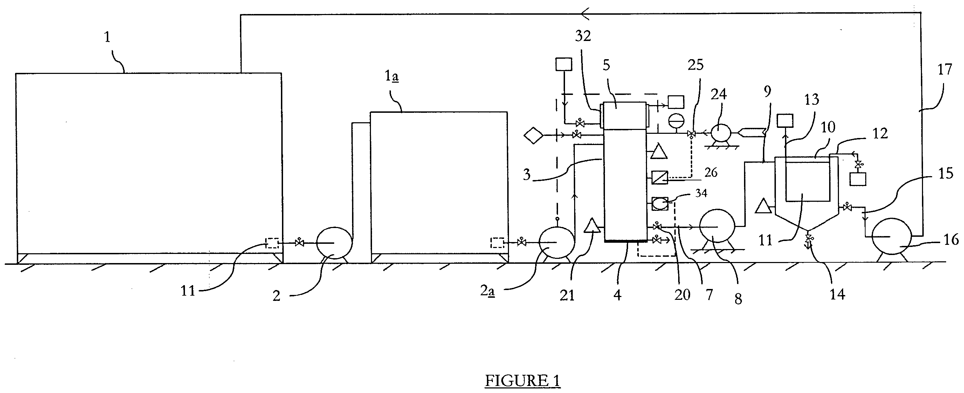

- la figure 1 représente schématiquement, en élévation, l'intégralité d'une installation permettant d'exploiter le procédé selon l'invention ;

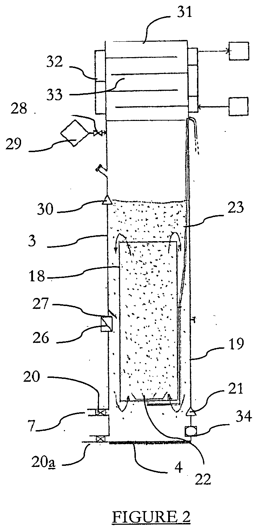

- la figure 2 représente schématiquement, en coupe axiale, le fermenteur-désucreur utilisé dans l'installation de la figure 1.

En se référant au dessin, on voit que l'on a désigné par 1 une cuve de vinification où l'on peut prélever le contenu de la cuve par une pompe 2, la sortie de la cure 1 étant équipée d'un filtre 11. La pompe 2 alimente un réservoir intermédiaire 1a, qui sert à envoyer en aval une quantité prédéterminée du vin de la cuve 1. La sortie du réservoir intermédiaire 1a est reliée à une pompe 2a, qui alimente le fermenteur-désucreur désigné par 3 dans son ensemble.

Le fermenteur-désucreur 3 a extérieurement une forme générale cylindrique ; il est fermé à sa base par une plaque chauffante 4 et il est surmonté d'un dévésiculeur 5, qui est fixé, à la partie supérieure du fermenteur-désucreur 3, par des fixations amovibles. Le dévésiculeur 5 comporte périphériquement une double enveloppe 32 alimentée en eau réfrigérée ; il comporte intérieurement une série de chicanes 33 et il permet de récupérer sensiblement la totalité du volume de la fraction de traitement chargée dans le fermenteur-désucreur 3.

La partie inférieure du fermenteur-désucreur 3 est reliée par la canalisation 7 à une pompe 8, qui alimente par la canalisation 9 un décanteur désigné par 10 dans son ensemble. Le décanteur 10 constitue un bac dans lequel se trouve un élément refroidisseur 11, dont le fluide refroidisseur, par exemple de l'eau, lui parvient par la canalisation 12 ; la sortie du liquide réfrigérant est effectuée par la canalisation 13. Le liquide, qui parvient au décanteur 10 par la canalisation 9, est refroidi par l'élément refroidisseur 11 et se décante dans le bac 10, le produit décanté formant une sorte de crème constituée par des levures que l'on peut évacuer par une vanne 14. Au niveau de la base de l'élément refroidisseur 11 est prévue une canalisation 15 munie d'une vanne et reliée à une pompe 16, dont la sortie 17 constitue un retour à la partie haute de la cuve de vinification 1.

Le fermenteur-désucreur 3 est délimité extérieurement par une enveloppe cylindrique 19, qui est destinée à recevoir l'aliquote de moût en cours de traitement provenant de la pompe 2a. Cette enveloppe cylindrique renferme intérieurement un conduit cylindrique de même axe, dont le diamètre a été défini de façon que la section droite du conduit cylindrique 18 ait une surface sensiblement égale à celle de l'anneau cylindrique compris entre l'enveloppe 19 et le conduit 18. L'enveloppe extérieure du fermenteur-désucreur 3 a été désignée par 19. A la base de l'enveloppe 19 se trouve une canalisation de vidange 20a équipée d'une vanne ; sensiblement au niveau de la canalisation 7, est disposé un détecteur de niveau bas 21.

La base du conduit 18 porte un dispositif d'insufflation d'air 22 ; l'alimentation en air du dispositif 22 est assurée par une pompe 24, dont la sortie est équipée d'une électrovanne 25 commandée notamment par les mesures faites par un réfractomètre 26. Le tube 23, qui remonte le long de la paroi de l'enveloppe 19, peut distribuer l'air, qui est dispersé à la base du conduit 18 par tout moyen approprié, par exemple par des éléments frittés.

Le réfractomètre 26 est porté par l'enveloppe 19 et est disposé dans l'espace annulaire 18/19 ; il est protégé du courant liquide, qui se déplace dans ledit espace annulaire, au moyen d'un déflecteur oblique 27, qui évite, sur l'optique, un dépôt de levures susceptible de perturber la mesure du taux de sucre faite par le réfractomètre 26, sans qu'une erreur intervienne par formation de bulles dans la zone de lecture.

Lorsque l'on démarre l'installation, il faut remplir le fermenteur-désucreur 3 en lui amenant une quantité pré-calculée du moût contenu dans la cuve de vinification 1 et l'introduction de cette aliquote de moût s'effectue par la vanne 28, qui est associée à un détecteur de niveau haut 29. Le niveau en cours d'exploitation est surveillé par un détecteur de niveau 30. Lorsque le moût a été introduit, on commence le chauffage au moyen de la plaque chauffante 4 et on introduit un anti-mousse alimentaire et les compléments nutritifs des levures, comme il est connu. Ceci correspond au démarrage de la phase de fermentation aérobie.

Une régulation de température est mise en place par action sur l'alimentation électrique de la plaque chauffante 4 ainsi qu'une régulation du débit d'air fourni par la pompe 24, on peut prévoir, par exemple, un débit d'air au nominal au voisinage de 300 L/mn. On préfère réguler la température au voisinage de 40°C.

Il est impératif que les levures aient la quantité nécessaire d'Oz, afin de ne pas produire d'alcool. Les levures étant des organismes dotés d'une grande capacité de multiplication, leur besoin en air est donc variable. C'est pourquoi on a prévu, selon l'invention, d'utiliser un photomètre, qui mesure en continu la croissance des levures et permet donc d'utiliser cette donnée pour assurer une régulation du débit d'air alimentant le fermenteur-désucreur.

Comme il résulte des indications précédemment données dans cette description, il est essentiel d'arrêter l'alimentation en air juste au moment où tout le sucre de la fraction en cours de traitement est consommé sans quoi il y aurait une oxydation des levures et il y aurait un parasitage inopportun du goût du vin. C'est la raison pour laquelle, selon l'invention, on a mis en oeuvre dans le fermenteur-désucreur 3, un réfractomètre 26, dont les mesures faites en continu ont été soigneusement protégées des perturbations pour assurer la fiabilité du procédé selon l'invention. L'ensemble des régulations susceptibles d'être mises en oeuvre avec le fermenteur-désucreur selon l'invention permet d'assurer toutes les fonctions requises par des automatismes gérables par un automate.

Au démarrage de la mise en oeuvre du fermenteur-désucreur, il est souhaitable d'atteindre 40°C le plus vite possible ; mais il est essentiel de ne pas excéder 60°C d'une part, pour préserver les levures et, d'autre part, pour ne pas faire caraméliser le sucre du jus de raisin sur la plaque chauffante 4. On prévoit donc de repérer la température de l'élément chauffant 4 avec au moins une sonde de température 34 située au voisinage de l'élément chauffant 4.

Le procédé, qui vient d'être décrit, permet de traiter un vin pour lequel D = 10 à 15 degrés alcooliques et d'obtenir un vin pour lequel D-d est compris entre 0,5 et 3 degrés alcooliques. Il peut être mis en oeuvre avec n'importe quelle levure sèche active (LSA) du commerce utilisée en œnologie pour réaliser une phase de désucrage. Lorsque l'on met en oeuvre un décanteur 10, les levures, qui sédimentent dans le décanteur durant la phase de clarification, représentent au moins 90% de la population levurienne, ce qui permet une forte valorisation du procédé selon l'invention.

## Revendications

1. Procédé de préparation d'un vin ayant de bonnes qualités organoleptiques et un faible degré d'alcool d, à partir d'un moût de raisin, dont le taux de sucre élevé conduirait, après un traitement de fermentation classique, à un vin de degré alcoolique potentiel D > d, ledit moût se trouvant dans une cuve de vinification à partir de laquelle on prélève une aliquote de moût dans un système de fermentation mettant en oeuvre des levures, ledit système comportant deux étapes de fermentation, dont l'une est aérobie et l'autre est anaérobie, le flux de sortie de la première étape alimentant la deuxième étape, qui fournit l'aliquote traitée et la renvoie dans la cuve de vinification pour y être mélangée avec le moût initial restant, **caractérisé en ce que**, dans un fermenteur-désucreur unique, on assure simultanément l'homogénéisation du milieu de fermentation et l'aération des levures, dont la multiplication en aérobie consomme du sucre sans produire d'éthanol, au moyen d'une circulation d'air associée à un flux circulant de l'aliquote provenant de la cuve de vinification, ladite homogénéisation s'effectuant avec un chauffage régulé, qui amène la température de l'aliquote à une valeur correspondant à une fermentation appropriée en mode aérobie jusqu'à une consommation complète ou quasi complète du sucre de l'aliquote de moût traité et que l'on transfère l'aliquote de traitement désucré depuis le fermenteur/désucreur vers un décanteur muni d'un moyen de refroidissement pour obtenir la clarification souhaitée du moût.

2. Procédé selon la revendication 1, **caractérisé en ce que** le flux circulant de l'aliquote de moût en cours de traitement et la circulation d'air associée sont effectués de bas en haut, parallèlement à la ligne moyenne du fermenteur-désucreur.

3. Procédé selon l'une des revendications 1 ou 2, **caractérisé en ce que** le chauffage du fermenteur-désucreur maintient, dans la fraction du moût en cours de traitement qu'il contient, une température sensiblement homogène comprise entre 30 et 45°C et de préférence, voisine de 40°C.

4. Procédé selon l'une des revendications 1 à 3, **caractérisé en ce que** l'aliquote du moût désucré réintroduite dans la cuve de vinification contient des levures, et provient du fermenteur-désucreur, afin d'ensemencer le moût restant dans ladite cuve pour assurer un redémarrage plus rapide de la vinification.

5. Procédé selon l'une des revendications 1 à 4, **caractérisé en ce que**, dans une phase de nettoyage ou de désinfection du fermenteur-désucreur, on utilise le chauffage du fermenteur-désucreur pour chauffer à ébullition une quantité d'eau de lavage qui, après condensation, est évacuée par une vanne de vidange.

6. Procédé selon l'une des revendications 1 à 5, **caractérisé en ce qu'**on mesure, de façon continue, au moyen d'un réfractomètre, le taux de sucre de l'aliquote de moût en cours de traitement, qui circule dans le fermenteur-désucreur, pour réguler automatiquement par action sur l'alimentation en air, le mode aérobie de la fermentation.

7. Procédé selon l'une des revendications 1 à 6, **caractérisé en ce qu'**on repère, par la mesure du taux de sucre dans le fermenteur-désucreur, le moment où tout le sucre de l'aliquote de traitement a été consommé par la fermentation aérobie et qu'on commande alors l'arrêt immédiat de l'entrée d'air ainsi que le chauffage dans le fermenteur-désucreur, pour écarter toute détérioration des qualités organoleptiques de ladite aliquote.

8. Procédé selon l'une des revendications 1 à 7, **caractérisé en ce qu'**on mesure la densité microbienne, donc la croissance des levures, dans l'aliquote de traitement, au moyen d'un photomètre et que l'on contrôle ainsi de manière complémentaire la fermentation dans le fermenteur-désucreur.

9. Procédé selon la revendication 1, **caractérisé en ce que** l'on recueille dans le décanteur tout ou partie des levures ayant sédimenté sous forme d'une crème dense concentrée en levures et on les réintroduit dans la cuve de vinification pour assurer, au moins partiellement, l'ensemencement de ladite cuve.

10. Procédé selon la revendication 9, **caractérisé en ce que** l'on améliore les qualités organoleptiques du vin à faible degré d'alcool en y injectant au moins une partie de la crème dense de levures récupérée dans le décanteur, après avoir traité lesdites levures, soit par traitement thermique, soit par digestion enzymatique partielle afin d'assurer l'élevage du vin.

## Patentansprüche

1. Verfahren zum Herstellen eines Weins mit guten organoleptischen Eigenschaften und einem niedrigen Alkoholgehalt d aus einem Traubenmost, dessen hoher Zuckergehalt nach einer herkömmlichen Gärungsbehandlung zu einem Wein mit einem potenziellen Alkoholgehalt D > d führt, wobei sich der Most in einem Weinbereitungsbehälter befindet, aus dem ein Mostaliquot in ein Gärungssystem entnommen wird, das Hefen einsetzt, wobei das System zwei Gärungsschritte aufweist, von denen einer aerob und der andere anaerob ist, wobei der Ausgangsstrom des ersten Schritts den zweiten Schritt versorgt, der das behandelte Aliquot bereitstellt und es in den Weinbereitungsbehälter zum dort Gemischtwerden mit dem verbleibenden ursprünglichen Most zurückführt, **dadurch gekennzeichnet, dass** in einer einzigen Gärungs-Entzuckerungsvorrichtung gleichzeitig die Homogenisierung des Gärungsmediums und die Belüftung der Hefen, deren aerobe Vermehrung Zucker verbraucht, ohne Ethanol zu produzieren, mittels einer Luftzirkulation sichergestellt wird, die mit einem zirkulierenden Strom des Aliquots verknüpft ist, das aus dem Weinbereitungsbehälter stammt, wobei die Homogenisierung mit einer regulierten Erwärmung erfolgt, die die Temperatur des Aliquots auf einen Wert bringt, der einer geeigneten Gärung in aerobem Modus bis zu einem vollständigen oder nahezu vollständigen Verbrauch des Zuckers des behandelten Mostaliquots entspricht, und dass das entzuckerte Behandlungsaliquot von der Gärungs-/Entzuckerungsvorrichtung in Richtung eines Dekanters überführt wird, der mit einem Kühlmittel zum Erhalten der gewünschten Klärung des Mosts versehen ist.

2. Verfahren nach Anspruch 1, **dadurch gekennzeichnet, dass** der zirkulierende Mostaliquotstrom während der Behandlung und die verknüpfte Luftzirkulation von unten nach oben parallel zu der Mittellinie der Gärungs-Entzuckerungsvorrichtung erfolgen.

3. Verfahren nach einem der Ansprüche 1 oder 2,
**dadurch gekennzeichnet, dass** das Erwärmen der Gärungs-Entzuckerungsvorrichtung in dem Anteil des Mosts während der Behandlung, den sie enthält, eine im Wesentlichen homogene Temperatur zwischen 30 und 45 °C und vorzugsweise nahe 40 °C aufrechterhält.

4. Verfahren nach einem der Ansprüche 1 bis 3,
**dadurch gekennzeichnet, dass** das Aliquot des entzuckerten Mosts, das in den Weinbereitungsbehälter wieder eingeführt wird, Hefen enthält und aus der Gärungs-Entzuckerungsvorrichtung stammt, um den Most, der in dem Behälter verbleibt, zum Sicherstellen eines schnelleren Neustarts der Weinbereitung zu beimpfen.

5. Verfahren nach einem der Ansprüche 1 bis 4,
**dadurch gekennzeichnet, dass** in einer Reinigungs- oder Desinfektionsphase der Gärungs-Entzuckerungsvorrichtung das Erwärmen der Gärungs-Entzuckerungsvorrichtung zum Erwärmen zu einem Sieden einer Menge von Waschwasser verwendet wird, das nach der Kondensation über ein Ablassventil entnommen wird.

6. Verfahren nach einem der Ansprüche 1 bis 5,
**dadurch gekennzeichnet, dass** der Zuckergehalt des Mostaliquots während der Behandlung, das in der Gärungs-Entzuckerungsvorrichtung zirkuliert, zum automatischen Regulieren des aeroben Modus der Gärung durch Beeinflussung der Luftversorgung mittels eines Refraktometers fortlaufend gemessen wird.

7. Verfahren nach 1 der Ansprüche 1 bis 6, **dadurch gekennzeichnet, dass** durch das Messen des Zuckergehalts in der Gärungs-Entzuckerungsvorrichtung der Zeitpunkt ermittelt wird, zu dem der gesamte Zucker des Behandlungsaliquots durch die aerobe Gärung verbraucht wurde, und dass dann der sofortige Stopp des Lufteintritts sowie der Erwärmung in der Gärungs-Entzuckerungsvorrichtung zum Vermeiden jeglicher Verschlechterung der organoleptischen Eigenschaften des Aliquots befohlen wird.

8. Verfahren nach einem der Ansprüche 1 bis 7,
**dadurch gekennzeichnet, dass** die mikrobielle Dichte, also das Hefewachstum, in dem Behandlungsaliquot mittels eines Photometers gemessen wird und so die Gärung in der Gärungs-Entzuckerungsvorrichtung komplementär gesteuert wird.

9. Verfahren nach Anspruch 1, **dadurch gekennzeichnet, dass** in dem Dekanter die gesamten oder ein Teil der Hefen, die sich ablagert haben, in Form einer dichten konzentrierten Creme aus Hefen gesammelt wird und sie wieder in den Weinbereitungsbehälter zum Sicherstellen mindestens teilweise der Beimpfung des Behälters eingeführt werden.

10. Verfahren nach Anspruch 9, **dadurch gekennzeichnet, dass** die organoleptischen Eigenschaften des Weins mit niedrigem Alkoholgehalt verbessert werden, indem mindestens ein Teil der dichten Creme aus Hefen, die in dem Dekanter gesammelt wird, in ihn injiziert wird, nachdem die Hefe entweder durch thermische Behandlung oder durch teilweise enzymatische Verdauung behandelt wurde, um den Ausbau des Weins sicherzustellen.

## Claims

1. A method for preparation of a wine having good organoleptic qualities and a low alcohol content d, from an initial must of grapes, whose high sugar content would lead, after a conventional fermentation process, to a wine with a potential alcoholic content D > d, said must of grapes being in a wine making vat from which an aliquot of must is taken in a fermentation system using yeasts, said system comprising two fermentation steps, one is aerobic and the other one is anaerobic, the output flow of the first step feeding the second step, which provides the processed aliquot and returns it in the said wine making vat to be mixed with the remaining initial must of grapes, **characterized in that** in a single fermenting-desugaring unit, a simultaneous homogenization of the fermentation medium and aeration of said yeasts takes place, the aerobic multiplication of the yeasts consuming sugar without producing ethanol, by means of an air circulation associated with a circulating flow of the aliquot providing from the wine making vat, said homogenization being achieved with controlled heating, which brings the temperature of the aliquot to a value corresponding to an appropriate fermentation in aerobic mode until a complete or almost complete consumption of the sugar of the aliquot of must being processed, the aliquot of must devoid of sugar being processed being transferred from the fermenting-desugaring unit to a settling vat equipped with a cooling means to obtain the desired clarification of the must.

2. The method of claim 1, **characterized in that** the circulating flow of the aliquot of must being processed and the associated air flow is performed from bottom to top, parallel to the average line of the fermenting-desugaring unit.

3. The method according to claim 1 or 2, **characterized in that** the heating of the fermenting-desugaring unit maintains, in the fraction of the must being processed that is contained therein, a substantially homogeneous temperature comprised between 30 and 45°C, and preferably, around 40°C.

4. The method according to one of claims 1 to 3, **characterized in that** the said aliquot of the must devoid of sugar reintroduced in the wine making vat contains yeasts, and comes from the fermenting-desugaring unit, in order to seed the initial must remaining in said wine making vat to ensure a faster restart of the wine making process.

5. The method according to one of claims 1 to 4, **characterized in that**, in a cleaning or disinfection phase of the fermenting-desugaring unit, the heating of the fermenting-desugaring unit is used to bring to a boil a quantity of wash water which, after condensation, is evacuated through a drain valve.

6. The method according to one of claims 1 to 5, **characterized in that** the sugar content of the aliquot of must being processed, which circulates in the fermenting-desugaring unit is continuously measured by means of a refractometer to regulate automatically the aerobic mode of fermentation.

7. The method according to one of claims 1 to 6, **characterized in that** the measurement of the sugar content of the aliquot being processed in the fermenting-desugaring unit is used to determine the moment when all the sugar of said aliquot of must has been consumed by the aerobic fermentation step and then to order the immediate shutdown of the said air circulation inflow and said controlled heating in the fermenting-desugaring unit, to avoid any deterioration of the organoleptic qualities of said aliquot.

8. The method according to one of claims 1 to 7, **characterized in that** the microbial density, and thus the growth of the yeasts, in the aliquot of must, is measured by means of a photometer, thus controlling in a complementary manner fermentation in the fermenting-desugaring unit.

9. The method according to one of claims 1 to 8, **characterized in that** all or part of the yeasts sedimented in the form of a dense laden cream concentrated in yeasts, are collected in the settling vat and reintroduced in the said wine making vat to ensure, at less partially, the seeding of said wine making vat.

10. The method according to claim 9, **characterized in that** the organoleptic qualities of wine with low alcohol content are improved by injecting at least a portion of the dense cream of yeasts recovered in said settling vat, after having processed said yeasts, either by heat treatment or by partial enzymatic digestion to ensure the breeding of the wine.
